# EUROPÄISCHE PATENTSCHRIFT

(11) **EP 3 287 077 B1**
(45) Veröffentlichungstag und Bekanntmachung des Hinweises auf die Patenterteilung: **12.12.2018**
(21) Anmeldenummer: 17184248.7
(22) Anmeldetag: 01.08.2017
(51) Int. Cl.: A61B 6/00, A61B 6/03, A61B 90/00

(54) **ANGULATIONSPLANUNG FÜR EINE DREIDIMENSIONALE ANGIOGRAPHIE**
ANGULATION PLANNING FOR THREE-DIMENSIONAL ANGIOGRAPHY
PLANIFICATION D'ANGULATION POUR UNE ANGIOGRAPHIE TRIDIMENSIONNELLE

(30) Priorität: 25.08.2016 DE 102016215970
(43) Veröffentlichungstag der Anmeldung: 28.02.2018
(73) Patentinhaber: Siemens Healthcare GmbH, 91052 Erlangen (DE)
(72) Erfinder: Redel, Thomas, 91099 Poxdorf (DE)

(56) Entgegenhaltungen:
- DE-A1-102006 011 242
- DE-A1-102014 210 591
- US-A1- 2007 036 264
- US-A1- 2011 044 525

## Beschreibung

Die Erfindung betrifft ein Verfahren zum Betreiben eines Röntgengerätes für ein Erstellen einer dreidimensionalen Angiographie eines Körpergefäßsegmentes oder Körpergefäßes.
Die Erfindung betrifft ebenfalls ein Röntgengerät für ein Erstellen einer dreidimensionalen Angiographie eines Körpergefäßsegments oder Körpergefäßes mit einer für das Verfahren geeigneten Recheneinrichtung.
Eine etablierte klinische Kenngröße ist die fraktionelle Flussreserve oder Fractional Flow Reserve (FFR), die beispielsweise mit einem Druckdraht gemessen werden kann. Der Druckdraht wird dabei in dem Körpergefäß oder Körpergefäßsegment an einer Stenose vorbeigeführt und dort der Druck distal zur Stenose bestimmt. Dieser distale Druck wird zur Berechnung der fraktionellen Flussreserve durch den proximalen Druck dividiert.
Es ist möglich, mittels eines dreidimensionalen Modells des Körpergefäßsegmentes oder Körpergefäßabschnittes, in welchem die Stenose enthalten ist, und weiteren Randbedingungen wie beispielsweise dem Blutfluss in Milliliter pro Sekunde durch das Körpergefäßsegment, den Druckverlauf über der Stenose mittels mathematischer Methoden der Fluiddynamik (computational fluid dynamics) zu berechnen und schließlich einen virtuellen Wert für die fraktionelle Flussreserve, einen virtuellen FFR-Wert, virtuell anhand des dreidimensionalen Modells zu berechnen. Derartige Methoden sind bekannt und beispielsweise in dem Artikel von Paul D. MORRIS et al.: ""Virtual" (Computed) Fractional Flow Reserve - Current Challenges and Limitations" in JACC: Cardiovascular Interventions, Vol. 8, No. 8, 2015, Seiten 1009 bis 1117, beschrieben. Auch weitere Berechnungsmethoden für einen virtuellen FFR-Wert sind bekannt, so zeigt z.B. DE 10 2014 210 591 eine Berechnung eines fluiddynamischen Kennwertes mittels einer 3D Rekonstruktion und einer Berechnung einer Mittellinie.

Grundsätzlich lassen sich die Ansätze zur virtuellen Berechnung der fraktionellen Flussreserve in zwei Gruppen aufteilen. Nichtinvasive Methoden, bei denen eine Geometrieinformation über das Körpergefäßsegment oder Körpergefäß mittels Computertomographie, Magnetresonanztomographie oder anderen Methoden gewonnen wird, und minimalinvasiven Methoden, bei welchen die Geometrieinformation im Herzkatheterlabor mittels Kontrastmittelinjektion in das Gefäß mit einer anschließenden Röntgenaufnahme gewonnen wird. In der Regel wird bei einem Patienten zunächst eine nichtinvasive Untersuchung mittels einer Computertomographie (CT) durchgeführt. Neben der diagnostischen Information über ein oder mehrere Gefäßquerschnitte des untersuchten Körpergefäßsegmentes oder Körpergefäßes kann dabei auch ein virtueller Wert für die fraktionelle Flussreserve berechnet werden, welcher im Folgenden als CT-FFR-Wert bezeichnet wird. Im Gegensatz dazu wird ein virtueller Wert für eine fraktionelle Flussreserve, welcher durch eine Angiographie zum Beispiel im Herzkatheterlabor ermittelt wird, im Folgenden als Angio-FFR-Wert bezeichnet.

Die CT-FFR-Methode, also das Berechnen des virtuellen FFR-Werts mittels eines CT hat, dabei den Vorteil, dass ein dreidimensionales Modell des gesamten Gefäßbaumes, in welchem das Körpergefäß oder das Körpergefäßsegment mit der Stenose verortet ist, vorhanden ist. Sie erlaubt auch eine gute Bestimmung der perfundierten Myokardmasse sowie des von dem Anteil der perfundierten Myokardmasse abgeleiteten Perfusionsflusses. Außerdem lassen sich auch zusätzliche Informationen wie beispielsweise eine Zusammensetzung der Stenose beziehungsweise des Plaque ermitteln. Nachteilig ist dabei die vergleichsweise geringe Ortsauflösung und damit eine ungenaue Geometriedarstellung der Stenosengeometrie.

Im Vergleich dazu hat die Angio-FFR-Methode, also das Berechnen eines virtuellen FFR-Wertes über eine Angiographie, den Vorteil einer guten Ortsauflösung, welche eine genaue Darstellung der Stenosengeometrie ermöglicht. Nachteilig ist dabei die Schätzung des Blutflusses über die Gefäßquerschnitte. Hier können bereits kleine Fehler große Auswirkungen haben. Auch ist die Schätzung des Blutflusses über die Kontrastmitteldynamik bei der Angio-FFR-Methode aufwändig und schwierig. Nachteilig ist des Weiteren, dass die Angio-FFR-Methode keine Informationen zu einem Zustand der Myokardmasse liefert, was beispielsweise wichtig ist, um eventuelle Vorschädigungen zu erkennen bei einer Behandlung berücksichtigen zu können. Überdies ist eine Geometrieinformation des gesamten Gefäßbaumes nur sehr schwer zu erlangen, was auch auf die üblicherweise bei Angiographien verwendeten relativ kleinen Detektoren zurückzuführen ist.

Der Erfindung liegt somit die Aufgabe zugrunde, ein Erstellen einer dreidimensionalen Angiographie zu verbessern, sodass insbesondere ein FFR-Wert schneller und genauer ermittelt werden kann.

Diese Aufgabe wird durch die Gegenstände der unabhängigen Patentansprüche gelöst. Vorteilhafte Ausführungsformen ergeben sich aus den abhängigen Patentansprüchen, der Beschreibung und den Figuren.

Die Erfindung betrifft ein Verfahren zum Betreiben eines Röntgengerätes für ein Erstellen einer dreidimensionalen Angiographie eines Körpergefäßsegmentes oder Körpergefäßes mit einer Reihe von Schritten. Insbesondere kann das Körpergefäß oder Körpergefäßsegment eine Koronare oder ein Segment einer Koronare umfassen.

Ein erster Schritt ist hier ein Bereitstellen einer dreidimensionalen Rekonstruktion des Körpergefäßsegmentes oder des Körpergefäßes, welche von einem anderen bildgebenden Gerät, insbesondere eines Röntgengerätes wie beispielsweise eines Computertomographiegerätes oder eines Magnetresonanztomographiegerätes aufgenommen wurde, an eine Recheneinrichtung des Röntgengerätes. Dies kann in Form eines Registrierdatensatzes erfolgen, der beispielsweise Daten über das dreidimensionale Modell, insbesondere mit Bilddaten und/oder Segmentierungsdaten und/oder Mittelliniendaten und/oder sonstigen Merkmalsdaten zur Berechnung eines CT-FFR-Wertes umfasst. Bei dem Körpergefäßsegment beziehungsweise Körpergefäß kann es sich um ein Herzkranzgefäßsegment beziehungsweise ein Herzkranzgefäßsegment handeln. Es kann sich bei dem Körpergefäßsegment oder Körpergefäß um ein erkranktes Körpergefäßsegment oder Körpergefäß handeln. Beispielsweise kann das Körpergefäßsegment oder Körpergefäß eine Stenose aufweisen.

Es kann, falls eine dreidimensionale Rekonstruktion des Körpergefäßes bereitgestellt wird, ein weiterer Schritt des Verfahrens ein Markieren des Körpergefäßsegmentes des Körpergefäßes in der dreidimensionalen Rekonstruktion sein. Dies kann bevorzugt durch eine Bedienperson erfolgen. Bei dem markierten oder bereitgestellten Körpergefäßsegment kann es sich um ein Körpergefäßsegment des Körpergefäßes handeln, in welchem eine Stenose vorhanden ist oder sonstige, insbesondere unerwünschte, Ablagerungen oder Veränderungen vorhanden sind.

Ein weiterer Schritt ist ein Berechnen einer Mittellinie durch das Körpergefäßsegment oder durch das markierte Körpergefäßsegment und ein Legen einer Rotationsachse durch die Mittellinie, wobei sowohl das Berechnen als auch das Legen der Rotationsachse mittels der Recheneinrichtung des Röntgengeräts erfolgt. Die Mittellinie folgt dabei einem Verlauf des Körpergefäßsegmentes entlang einer Mitte des Körpergefäßsegmentes und somit beispielsweise einem Blutfluss durch das Körpergefäßsegment. Die Rotationsachse kann als Gerade die Mittellinie beispielsweise derart annähern, dass ein Fehler zwischen Mittellinie und Rotationsachse, beispielsweise ein Abstandsquadrat, minimiert wird.

Die Mittellinie kann hier lokal in einem vorbestimmten Bereich, insbesondere dem Bereich der erkannten Stenose berechnet werden. Dies kann in einer bereits segmentierten dreidimensionalen Rekonstruktion des Körpergefäßsegmentes erfolgen, aber auch auf Bilddaten. In diesem Fall ist zumindest eine lokale Segmentierung im Bereich des Körpergefäßsegmentes oder markierten Körpergefäßsegmentes, also der Stenose, notwendig. Unter einer Segmentierung kann hier die Zuordnung eines Pixels oder eines Voxels zu einem Körperteil verstanden werden.

Ein nächster Schritt ist ein Registrieren der dreidimensionalen Rekonstruktion zu dem Röntgengerät. Dies kann auch durch ein Registrieren des Registrierdatensatzes der dreidimensionalen Rekonstruktion zu einem von dem Röntgengerät aufgenommenen Planungsdatensatz des Körpergefäßes oder Körpergefäßsegmentes erfolgen. Das Registrieren ist hier im Sinne einer Bildregistrierung zu verstehen, bei welcher die dreidimensionale Rekonstruktion in einen definierten, eindeutig bestimmten räumlichen Bezug zu dem Röntgengerät beziehungsweise der Registrierdatensatz mit dem Planungsdatensatz in einen definierten, eindeutig bestimmten Bezug gesetzt wird, um das Körpergefäß oder Körpergefäßsegment in der dreidimensionalen Rekonstruktion in räumliche Übereinstimmung mit dem Körpergefäßsegment oder Körpergefäß in dem aufgenommenen Planungsdatensatz beziehungsweise in dem Röntgengerät zu bringen. Methoden zur automatischen oder semiautomatischen Bildregistrierung in drei Dimensionen oder zwei Dimensionen sowie Mischungen von zwei- und dreidimensionalen Daten sind bekannt. Damit wird ermöglicht, das Röntgengerät oder Teile des Röntgengeräts wie beispielsweise eine Aufnahmeeinheit in eine vorgegebene räumliche Relation zu dem (insbesondere markierten) Körpergefäßsegment der dreidimensionalen Rekonstruktion zu bringen, was für das weitere Verfahren wichtig ist.

Ein nächster Schritt ist ein Bewerten der Eignung von zumindest einem Aufnahmewinkelpaar mit einem ersten und einem zweiten Aufnahmewinkel für das Erstellen der dreidimensionalen Angiographie anhand eines Bewertungskriteriums mittels der Recheneinrichtung. Das Bewerten kann also ein oder mehrere Aufnahmewinkelpaare betreffen. Es kann auch wiederholt durchgeführt werden. Das Bewerten der Eignung eines Aufnahmewinkelpaares kann auch ein Bewerten der Eignung von dem ersten und/oder dem zweiten Aufnahmewinkel an sich umfassen. Das Bewerten der Eignung eines Aufnahmewinkelpaares kann insbesondere auch als ein Bewerten eines ersten und eines zweiten Aufnahmewinkels in wechselseitiger Abhängigkeit verstanden werden.

Die dreidimensionale Angiographie wird dabei mit einer ersten Aufnahme des Körpergefäßes oder Körpergefäßsegmentes durch das Röntgengerät aus dem ersten Aufnahmewinkel und einer zweiten Aufnahme des Körpergefäßes durch das Röntgengerät aus dem zweiten Aufnahmewinkel erzeugt. Die bewerteten Aufnahmewinkel stehen dabei bevorzugt jeweils senkrecht zu der Rotationsachse. Der Begriff senkrecht umfasst hier insbesondere auch "im Wesentlichen senkrecht", also eine Abweichung des rechten Winkels um wenige Grad, beispielsweise um weniger als 15°, weniger als 10° oder weniger als 5°. Dadurch kann die dreidimensionale Angiographie mit besonders großer Genauigkeit erzeugt werden. Die Aufnahmewinkel oder Aufnahmewinkelpaare, welche bewertet werden, können dabei zufällig vorausgewählt werden. Es kann auch vorgesehen sein, sämtliche Aufnahmewinkel oder Aufnahmewinkelpaare, insbesondere sämtliche durch das Röntgengerät anfahrbare Aufnahmewinkel oder Aufnahmewinkelpaare zu bewerten, insbesondere sämtliche anfahrbare Aufnahmewinkel oder Aufnahmewinkelpaare, welche senkrecht zu der Rotationsachse stehen.

Ein nächster Schritt ist schließlich ein Auswählen eines des zumindest einen bewerteten Aufnahmewinkelpaares, das heißt des bewerteten Aufnahmewinkelpaares oder eines der bewerteten Aufnahmewinkelpaare, zum Erstellen der dreidimensionalen Angiographie. Das Auswählen erfolgt in Abhängigkeit eines Ergebnisses des Bewertens. Somit kann hier das Aufnahmewinkelpaar ausgewählt werden, welches bei dem Bewerten das beste Ergebnis erzielt hat. Insbesondere kann die Auswahl des Aufnahmewinkelpaares auch schrittweise erfolgen, beispielsweise erst eine Auswahl des ersten Aufnahmewinkels erfolgen und sodann in Abhängigkeit von dem gewählten ersten Aufnahmewinkel ein zweiter Aufnahmewinkel ausgewählt werden. Dabei kann das Bewerten des zweiten Aufnahmewinkels auch getrennt von dem Bewerten des ersten Aufnahmewinkels erfolgen und insbesondere nach der Auswahl des ersten Aufnahmewinkels. Es kann auch vorgesehen sein, ein oder das Aufnahmewinkelpaar nur auszuwählen, falls das Ergebnis des Bewertens ein vorgegebenes Mindestergebnis erreicht, beispielsweise eine vorgegebene Mindestqualität. Dies ist insbesondere vorteilhaft, falls nur ein Aufnahmewinkelpaar bewertet wird.

Das hat den Vorteil, dass die bereits vorhandenen Informationen über das Körpergefäß oder Körpergefäßsegment, welche in der dreidimensionalen Rekonstruktion des Körpergefäßsegmentes oder Körpergefäßes enthalten sind, für die Planung der Angiographie beziehungsweise der für die dreidimensionale Angiographie genutzten Winkel oder Angulationen genutzt werden, um eine Angiographie mit gegenüber bekannten Verfahren verbesserter Qualität und damit auch insbesondere genauere Werte für einen virtuellen FFR-Wert zu erzielen, welcher basierend auf der verbesserten Angiographie berechnet wird. Dabei wird nicht nur eine Bildqualität erhöht, sondern auch Zeit gespart, da eine Bedienperson in der Wahl der Aufnahmewinkel unterstützt wird und eine geringere Anzahl von Aufnahmen für eine Angiographie von hinreichender Qualität erforderlich ist. Auch wird somit eine Strahlenexposition des Patienten minimiert, die für die Berechnung des virtuellen FFR-Werts erforderlich ist.

In einer vorteilhaften Ausführungsform ist vorgesehen, dass das Bewertungskriterium einen Grad einer Überlagerung des Körpergefäßsegmentes oder markierten Körpergefäßsegmentes mit zumindest einem weiteren Körpergefäßsegment oder weiteren Segmenten des Körpergefäßes auf der jeweiligen Aufnahme des einen oder markierten Körpergefäßsegmentes umfasst. Dabei wird ein geringer Überlagerungsgrad besser bewertet als ein hoher Überlagerungsgrad. Der Überlagerungsgrad oder das Überlagerungsmaß kann dabei beispielsweise derart bestimmt werden, dass für jede Angulation oder jeden Aufnahmewinkel aus der dreidimensionalen Rekonstruktion eine Projektion erstellt wird und der Anteil der Pixel bestimmt wird, auf welche zwei oder mehr der entsprechenden Körpergefäßsegmente projiziert werden. Dieses Maß ist dann zu minimieren. Das hat den Vorteil, dass gerade das eine beziehungsweise das markierte Körpergefäßsegment, welches für die Angiographie von besonderem Interesse ist, beispielsweise aufgrund einer vorhandenen Stenose, besonders gut in der Angiographie sichtbar ist und damit besonders genau quantifiziert werden kann. Es erleichtert auch eine spätere Segmentierung der Bilddaten für die Angiographie, also ein Zuordnen von Pixeln oder Voxeln zu dem Körpergefäß (beziehungsweise Körpergefäßsegment) oder anderen Strukturen.

In einer weiteren vorteilhaften Ausführungsform ist dabei vorgesehen, dass das Bewertungskriterium einen Grad einer peripheren Überlagerung zumindest eines weiteren Körpergefäßsegmentes untereinander, das heißt mit sich, oder mehrerer unterschiedlicher weiterer Körpergefäßsegmente untereinander auf der jeweiligen Aufnahme des einen oder markierten Körpergefäßsegmentes umfasst. Auch hierbei wird ein geringer Überlagerungsgrad besser bewertet als ein hoher Überlagerungsgrad. Insbesondere kann dabei der Grad der peripheren Überlagerung bei dem Ergebnis des Bewertens weniger berücksichtigt werden als der Grad der Überlagerung für das eine oder markierte Körpergefäßsegment. In diesem Fall umfasst das Bewertungskriterium also den Grad der Überlagerung, welcher im letzten Absatz beschrieben ist sowie den Grad der hier beschriebenen peripheren Überlagerung. Neben den bereits im letzten Absatz genannten Vorteilen kann das markierte beziehungsweise eine Körpergefäßsegment, in welchem beispielsweise die Stenose vorhanden ist, mit einer besonders großen Genauigkeit in der Angiographie erfasst werden, sodass insgesamt für beispielsweise die Berechnung eines Angio-FFR-Wertes eine optimale Angulations- oder Aufnahmewinkelplanung erreicht werden kann.

In einer weiteren vorteilhaften Ausführungsform ist vorgesehen, dass das Bewertungskriterium eine Anfahrbarkeit des entsprechenden Aufnahmewinkels oder der entsprechenden Aufnahmewinkel durch das Röntgengerät umfasst und ein nicht durch das Röntgengerät anfahrbarer Aufnahmewinkel nicht zum Erstellen der dreidimensionalen Angiographie ausgewählt werden kann. Insbesondere kann dabei ein mit einem geringeren Bewegungsaufwand, also beispielsweise mit einer kleineren Bewegung und/oder in einer kürzeren Zeit, anfahrbares Aufnahmewinkelpaar besser bewertet werden, als ein mit einem höheren Bewegungsaufwand anfahrbares Aufnahmewinkelpaar. Da es sich ganz allgemein bei dem Röntgengerät beispielsweise um ein C-Bogen Röntgengerät handeln kann, kann in diesem Fall eine Anfahrbarkeit durch einen C-Bogen des Röntgengeräts berücksichtigt werden. Die Anfahrbarkeit kann beispielsweise anhand von Tabellen in dem Röntgengerät hinterlegt sein. Das hat den Vorteil, dass eine Bedienzeit und/oder Aufnahmezeit reduziert wird und eine Bedienperson durch vermeintlich vorteilhafte Angulationen oder Aufnahmewinkel, welche aber nicht an dem Röntgengerät realisierbar sind, nicht in die Irre geführt wird. Da somit auch ein Anfahren eines weniger geeigneten Ersatz-Aufnahmewinkels in der Nähe des nichtanfahrbaren Aufnahmewinkels durch das Röntgengerät von vornherein verhindert wird, wird damit auch die Genauigkeit der Angiographie erhöht.

In einer weiteren vorteilhaften Ausführungsform ist vorgesehen, dass die Aufnahmewinkel in einer Ebene senkrecht, insbesondere im Wesentlichen senkrecht, zu der Rotationsachse einen vorgegebenen oder vorgebbaren Zwischenwinkel einschließen und das Bewertungskriterium den Zwischenwinkel umfasst. Dabei wird ein Aufnahmewinkelpaar mit einem größeren Zwischenwinkel besser bewertet als ein Aufnahmewinkelpaar mit einem kleineren Zwischenwinkel. Der Zwischenwinkel ist dabei der Betrag des kleineren Differenzwinkels zwischen den Aufnahmewinkeln, kann also nur zwischen 0° und 90° liegen. Das hat den Vorteil, dass die dreidimensionale Angiographie aus der zweidimensionalen ersten und der zweidimensionalen zweiten Aufnahme aus dem ersten und zweiten Aufnahmewinkel in ihrer Qualität verbessert ist.

In einer anderen vorteilhaften Ausführungsform ist vorgesehen, dass das Bewertungskriterium eine zu erwartende Strahlenbelastung eines Patienten, für dessen Körpergefäßsegment oder markiertes Körpergefäßsegment die Angiographie erstellt wird, umfasst. Dabei wird eine geringe Strahlenbelastung besser bewertet als eine hohe Strahlenbelastung. Auch hier kann die zu erwartende Strahlenbelastung in Form einer Tabelle für die unterschiedlichen Aufnahmewinkel in dem Röntgengerät hinterlegt sein oder gemäß einem Modell berechnet werden. Das hat den Vorteil, dass eine Gesundheit des Patienten geschont oder besser erhalten wird.

In einer weiteren Ausführungsform ist vorgesehen, dass das Bewertungskriterium eine zu erwartende Bildqualität der Aufnahme umfasst, insbesondere ein zu erwartendes Signal-zu-Rauschverhältnis (signal-to-noise-ratio, SNR) und/oder eine zu erwartende Bildschärfe. Dabei wird eine hohe Bildqualität besser bewertet als eine geringe Bildqualität. Auch dies hat den Vorteil, dass die dreidimensionale Angiographie verbessert wird und entsprechend Folgeberechnungen wie beispielsweise der Angio-FFR-Wert mit einer höheren Genauigkeit bestimmt werden kann.

In einer weiteren Ausführungsform ist vorgesehen, dass das Bewertungskriterium eine für die Aufnahmen veränderbare Tischposition eines Patiententisches des Röntgengeräts umfasst. Dabei wird ein Aufnahmewinkelpaar ohne eine Veränderung beziehungsweise mit einer geringeren Veränderung der Tischposition besser bewertet als ein Aufnahmewinkelpaar mit einer Veränderung beziehungsweise mit einer größeren Veränderung der Tischposition. Da zusätzliche Bewegungen stets auch eine zusätzliche Ungenauigkeit beziehungsweise einen zusätzlichen Rechenaufwand, um eine entsprechende Ungenauigkeit zu vermeiden, erfordern, ist eine Berücksichtigung einer gegebenenfalls erforderlichen Veränderung der Tischposition bei der Bewertung der Aufnahmewinkel beziehungsweise des Aufnahmewinkelpaares vorteilhaft, um eine verbesserte Genauigkeit für die dreidimensionale Angiographie zu erreichen.

Jeweilige in den unterschiedlichen Ausführungsformen genannten unterschiedlichen Komponenten oder Teile des Bewertungskriteriums können dabei auch gemeinsam mit einer oder mehreren anderen Komponenten des Bewertungskriteriums berücksichtigt und unterschiedlich gewichtet werden. Insbesondere kann eine Wichtung entsprechend der hier genannten Reihenfolge mit den zuerst genannten Komponenten als wichtigste Komponenten erfolgen: Grad der Überlagerung des Körpergefäßsegmentes mit zumindest einem weiteren Körpergefäßsegment, Grad der peripheren Überlagerung, Anfahrbarkeit des entsprechenden Aufnahmewinkels oder entsprechenden Aufnahmewinkel, Zwischenwinkel zwischen den Aufnahmewinkeln, die zu erwartende Strahlenbelastung des Patienten, die zu erwartende Bildqualität der Aufnahme, und die für die Aufnahmen veränderbare Tischposition des Patiententisches. Damit kann in besonders vorteilhafter Weise eine zweckdienliche dreidimensionale Angiographie erstellt werden.

In einer weiteren Ausführungsform ist vorgesehen, dass die Tischposition des Patiententisches jeweils für ein Aufnahmewinkelpaar festgelegt wird und insbesondere sich das eine oder das markierte Körpergefäßsegment durch das Festlegen in einem Isozentrum des Röntgengerätes befindet. Das hat den Vorteil, dass die Veränderung der Tischposition als zusätzlicher zu berücksichtigender Parameter von vornherein ausgeschlossen wird und somit das Erstellen der dreidimensionalen Angiographie erleichtert beziehungsweise das Ergebnis verbessert wird. Die Anordnung des einen oder markierten Körpergefäßsegmentes in einem Isozentrum des Röntgengerätes ist hier besonders vorteilhaft.

In einer weiteren vorteilhaften Ausführungsform ist vorgesehen, dass der in der Ebene senkrecht zu der Rotationsachse von den Aufnahmewinkeln eingeschlossene Zwischenwinkel zwischen 25° und 90° beträgt, bevorzugt zwischen 30° und 90°. Das hat den Vorteil, dass die Qualität der dreidimensionalen Angiographie eine vorgegebene Mindestqualität erreicht.

In einer anderen vorteilhaften Ausführungsform ist vorgesehen, dass das Auswählen des Aufnahmewinkelpaares oder des ersten und des zweiten Aufnahmewinkels automatisch durch die Recheneinrichtung des Röntgengerätes erfolgt. Es kann auch alternativ oder ergänzend vorgesehen sein, dass automatisch durch die Recheneinrichtung des Röntgengerätes eine Vorauswahl oder ein Vorauswählen erfolgt, sodass in einem nächsten Schritt ein Auswählen durch eine Bedienperson aus einer reduzierten Anzahl an unterschiedlichen Aufnahmewinkelpaaren erfolgt. Dabei kann der Bedienperson insbesondere für die unterschiedlichen Aufnahmewinkelpaare oder Aufnahmewinkel ein Ergebnis des Bewertens angezeigt werden. Das hat den Vorteil, dass auf besonders einfache und schnelle Weise viele Aufnahmewinkel oder Aufnahmewinkelpaare bewertet und somit berücksichtigt werden können, sodass am Ende tatsächlich der bestmögliche Aufnahmewinkel oder das bestmögliche Aufnahmewinkelpaar für das Erstellen der dreidimensionalen Angiographie ausgewählt und im Folgenden verwendet werden kann.

Die Erfindung betrifft auch ein Röntgengerät für ein Erstellen einer dreidimensionalen Angiographie eines Körpergefäßsegments oder Körpergefäßes mit einer Recheneinrichtung, an welche eine dreidimensionale Rekonstruktion des Körpergefäßsegments oder Körpergefäßes bereitstellbar ist. Dabei ist durch oder mittels der Recheneinrichtung eine Mittellinie durch das Körpergefäßsegment berechenbar sowie eine Rotationsachse durch die Mittellinie legbar. Die dreidimensionale Rekonstruktion ist dabei auch durch die Recheneinrichtung zu dem Röntgengerät registrierbar. Ferner ist die Recheneinrichtung ausgelegt, die Eignung von zumindest einem Aufnahmewinkelpaar mit einem ersten und einem zweiten Aufnahmewinkel für das Erstellen der dreidimensionalen Angiographie anhand eines Bewertungskriteriums zu bewerten. Dabei stehen die bewerteten Aufnahmewinkel jeweils senkrecht zu der Rotationsachse. Die Recheneinrichtung ist weiterhin ausgelegt, eines des zumindest einen Aufnahmewinkel-Paares mit einem der ersten und einem der zweiten Aufnahmewinkel zum Erstellen der dreidimensionalen Angiographie in Abhängigkeit eines Ergebnisses des Bewertens auszuwählen oder vorauszuwählen.

Vorteile und vorteilhafte Ausführungsformen des Röntgengerätes entsprechen hier Vorteilen und vorteilhaften Ausführungsformen des oben beschriebenen Verfahrens.

Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind somit auch Ausführungen und Merkmalskombinationen als offenbart anzusehen, die somit nicht alle Merkmale eines ursprünglich formulierten abhängigen oder unabhängigen Anspruchs aufweisen.

Ein Ausführungsbeispiel der Erfindung wird nachfolgend anhand schematischer Zeichnungen näher erläutert. Dabei zeigen:
- FIG 1: ein schematisches Flussdiagramm einer beispielhaften Ausführungsform eines Verfahrens zum Betreiben eines Röntgengeräts für ein Erstellen einer dreidimensionalen Angiographie; und
- FIG 2: eine beispielhafte Darstellung eines Körpergefäßsegmentes mit einer Stenose und einer Rotationsachse.

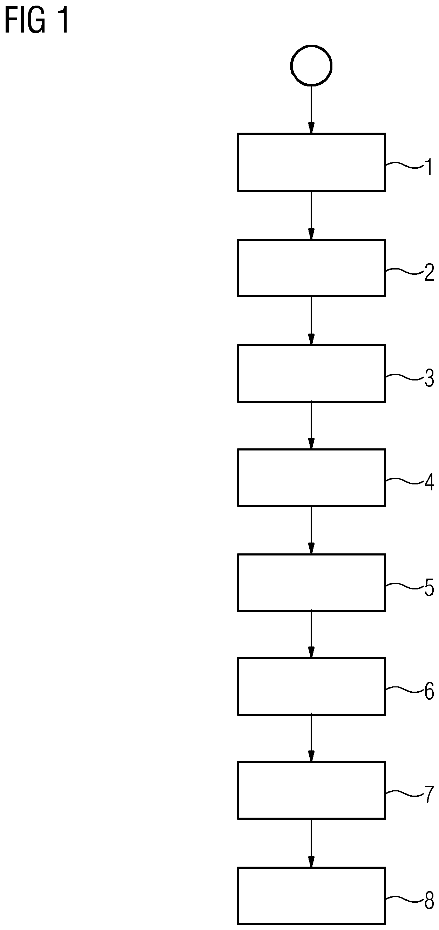

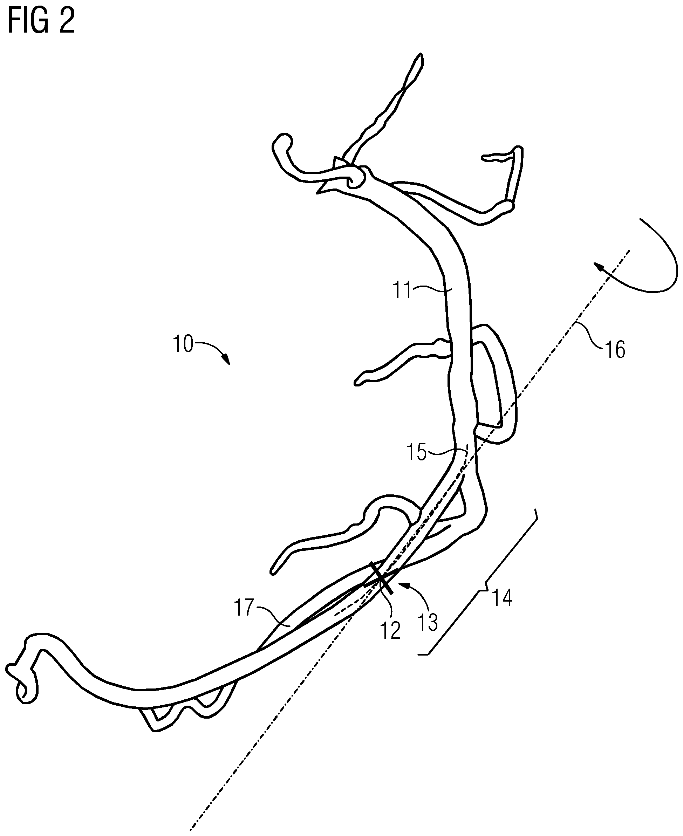

In der in FIG 1 dargestellten beispielhaften Ausführungsform eines Verfahrens zum Betreiben eines Röntgengeräts für ein Erstellen einer dreidimensionalen Angiographie eines Körpergefäßes erfolgt zunächst ein Bereitstellen 1 einer dreidimensionalen Rekonstruktion 10 (FIG 2) des Körpergefäßes 11 (FIG 2) von einem anderen bildgebenden Gerät, vorliegend einem Computertomographen. Die dreidimensionale Rekonstruktion des Körpergefäßes wird dabei an eine Recheneinrichtung des Röntgengeräts bereitgestellt, vorliegend in Form eines noch zu dem Röntgengerät zu registrierenden Registrierdatensatzes. In einem nächsten Schritt erfolgt hier ein Markieren 2 eines Körpergefäßsegmentes des Körpergefäßes in der dreidimensionalen Rekonstruktion durch eine Bedienperson. Die Bedienperson kann hier beispielsweise ein Körpergefäßsegment 14 (FIG 2) des Körpergefäßes, in welchem eine Stenose 13 (FIG 2) vorhanden ist, markieren. Als nächstes erfolgt ein Berechnen 3 einer Mittellinie 15 (FIG 2) durch das markierte Körpergefäßsegment mittels der Recheneinrichtung. Dabei folgt die Mittellinie hier dem Verlauf des Körpergefäßes im Bereich der Stenose. In einem weiteren Schritt erfolgt ein Legen 4 einer Rotationsachse 16 (FIG 2) durch die Mittellinie durch die Recheneinrichtung, sodass die Recheneinrichtung im folgenden Aufnahmewinkel oder Angulationen um die Rotationsachse berechnen kann, die das markierte Gefäßsegment in seinem Längsverlauf zeigen.

In einem nächsten Schritt erfolgt nun ein Registrieren 5 des Registrierdatensatzes und somit der dreidimensionalen Rekonstruktion zu einem von dem Röntgengerät für das Erstellen der dreidimensionalen Angiographie aufgenommenen Planungsdatensatz des Körpergefäßes und somit zu dem Röntgengerät. Damit kann der Registrierdatensatz und somit Merkmale oder Informationen aus der dreidimensionalen Rekonstruktion für das Erstellen der dreidimensionalen Angiographie durch das Röntgengerät, hier ein Auswählen 7 eines geeigneten Aufnahmewinkels oder zweier geeigneter Aufnahmewinkel als Aufnahmewinkelpaar durch das Röntgengerät erfolgen.

Als weiterer Schritt erfolgt ein Bewerten 6 der Eignung von zumindest einem Aufnahmewinkelpaar mit einem ersten und einem zweiten Aufnahmewinkel, vorliegend eine Vielzahl von solchen Aufnahmewinkelpaaren, oder von zumindest einem ersten und/oder von zumindest einem zweiten Aufnahmewinkel für das Erstellen der dreidimensionalen Angiographie anhand eines Bewertungskriteriums mittels der Recheneinrichtung. Die bewerteten Aufnahmewinkel stehen jeweils senkrecht zu der Rotationsachse. Im gezeigten Beispiel werden hier vorliegend sämtliche mögliche senkrecht zur Rotationsachse stehenden Aufnahmewinkel bewertet, um das am besten für die dreidimensionale Angiographie geeignete Aufnahmewinkelpaar zu ermitteln.

Das Bewertungskriterium umfasst im gezeigten Beispiel einen Grad einer Überlagerung des markierten Körpergefäßsegmentes mit zumindest einem weiteren Körpergefäßsegment des Körpergefäßes auf der jeweiligen Aufnahme des markierten Körpergefäßsegmentes. Da gerade im Bereich der Stenose eine überlagerungsfreie Aufnahme wichtig ist, erhält vorliegend dieses Bewertungskriterium im Vergleich zu den weiteren im Folgenden genannten Komponenten des Bewertungskriteriums die größte Gewichtung bei einem Ergebnis des Bewertens.

Des Weiteren umfasst das Bewertungskriterium vorliegend einen Grad einer peripheren Überlagerung zumindest eines weiteren Körpergefäßsegmentes untereinander sowie eine Anfahrbarkeit der entsprechenden Aufnahmewinkel durch das Röntgengerät. Damit werden nicht oder schlecht anfahrbare Aufnahmewinkel oder Aufnahmewinkelpaare aussortiert und auch die Überlagerung für die weiteren Gefäßsegmente, in welchen keine Stenose vorhanden ist, in den Aufnahmen minimiert.

Somit kann in einem weiteren Schritt ein Auswählen 7 eines bewerteten Aufnahmewinkelpaares der dreidimensionalen Angiographie erfolgen. Dies erfolgt in Abhängigkeit eines Ergebnisses des Bewertens, sodass das Aufnahmewinkelpaar mit dem besten Ergebnis ausgewählt wird. Dies kann automatisch erfolgen. Vorliegend erfolgt das Auswählen 7 jedoch teilautomatisch, das heißt es erfolgt zunächst eine automatische Vorauswahl, aus welcher sodann durch eine Bedienperson das eine Aufnahmewinkelpaar, welches für das Erstellen der dreidimensionalen Angiographie genutzt werden soll, ausgewählt wird.

Schließlich erfolgt in einem letzten Schritt vorliegend das Erstellen 8 der dreidimensionalen Angiographie des Körpergefäßes mit dem markierten Körpergefäßsegment durch das Röntgengerät mittels je einer Aufnahme aus dem ersten und dem zweiten Aufnahmewinkel des ausgewählten Aufnahmewinkelpaares.

FIG 2 zeigt eine beispielhafte Darstellung eines Körpergefäßsegmentes mit einer Stenose und einer Rotationsachse. Die dreidimensionale Rekonstruktion 10 des Körpergefäßes 11 weist vorliegend an einer Position 12 eine Stenose 13 auf. Entsprechend wurde vorliegend ein Körpergefäßsegment 14 durch eine Bedienperson markiert und durch eine Recheneinrichtung des Röntgengeräts eine Mittellinie 15 durch das Körpergefäßsegment 14 berechnet. Des Weiteren wurde eine Rotationsachse 16 durch die Mittellinie 15 gelegt, zu welcher die anschließend anhand des Bewertungskriteriums durch die Recheneinrichtung bewerteten Aufnahmewinkel senkrecht stehen. Auch ein weiteres Körpergefäßsegment 17, welches sich in der gezeigten Ansicht teilweise hinter dem Körpergefäßsegment 14 befindet und sich somit in einer Aufnahme mit dem Körpergefäßsegment 14 überlagern könnte, ist hier vorhanden.

Damit können die Informationen aus der dreidimensionalen Rekonstruktion 10 für das Erstellen 8 (FIG 1) der dreidimensionalen Angiographie genutzt werden und eine optimierte dreidimensionale Angiographie erstellt werden.

## Patentansprüche

1. Verfahren zum Betreiben eines Röntgengerätes für ein Erstellen (8) einer dreidimensionalen Angiographie eines Körpergefäßsegmentes (14), mit den Schritten:
a) Bereitstellen (1) einer dreidimensionalen Rekonstruktion (10) des Körpergefäßsegmentes (14) an eine Recheneinrichtung des Röntgengerätes;
b) Berechnen (3) einer Mittellinie (15) durch das Körpergefäßsegment (14) und Legen (4) einer Rotationsachse (16) durch die Mittellinie (15) mittels der Recheneinrichtung;
c) Registrieren (5) der dreidimensionalen Rekonstruktion (10) zu dem Röntgengerät;
d) Bewerten (6) der Eignung von zumindest einem Aufnahmewinkel-Paar mit einem ersten und einem zweiten Aufnahmewinkel für das Erstellen (8) der dreidimensionalen Angiographie anhand eines Bewertungskriteriums mittels der Recheneinrichtung;
e) Auswählen (7) eines des zumindest einen bewerteten Aufnahmewinkel-Paares zum Erstellen (8) der dreidimensionalen Angiographie in Abhängigkeit eines Ergebnisses des Bewertens.

2. Verfahren nach Anspruch 1,
**dadurch gekennzeichnet, dass**
das Bewertungskriterium einen Grad einer Überlagerung des Körpergefäßsegmentes (14) mit zumindest einem weiteren Körpergefäßsegment (17) auf der jeweiligen Aufnahme des einen Körpergefäßsegmentes (14) umfasst, und ein geringer Überlagerungsgrad besser bewertet wird als ein hoher Überlagerungsgrad.

3. Verfahren nach einem der vorhergehenden Ansprüche,
**dadurch gekennzeichnet, dass**
das Bewertungskriterium einen Grad einer peripheren Überlagerung zumindest eines weiteren Körpergefäßsegmentes (17) untereinander auf der jeweiligen Aufnahme des einen Körpergefäßsegmentes (14) umfasst, und ein geringer Überlagerungsgrad besser bewertet wird als ein hoher Überlagerungsgrad, wobei insbesondere der Grad der peripheren Überlagerung bei dem Ergebnis des Bewertens weniger berücksichtigt wird als der Grad der Überlagerung für das eine Körpergefäßsegment (14).

4. Verfahren nach einem der vorhergehenden Ansprüche,
**dadurch gekennzeichnet, dass**
das Bewertungskriterium eine Anfahrbarkeit des entsprechenden Aufnahmewinkels durch das Röntgengerät umfasst, und ein nicht durch das Röntgengerät anfahrbarer Aufnahmewinkel nicht zum Erstellen (8) der dreidimensionalen Angiographie ausgewählt werden kann, sowie insbesondere ein mit einem geringeren Bewegungsaufwand anfahrbares Aufnahmewinkel-Paar besser bewertet wird als ein mit einem höheren Bewegungsaufwand anfahrbares Aufnahmewinkel-Paar.

5. Verfahren nach einem der vorhergehenden Ansprüche,
**dadurch gekennzeichnet, dass**
die Aufnahmewinkel in einer Ebene senkrecht zu der Rotationsachse (16) einen vorgegebenen Zwischenwinkel einschließen und das Bewertungskriterium den Zwischenwinkel umfasst, und ein Aufnahmewinkel-Paar mit einem größeren Zwischenwinkel besser bewertet wird als ein Aufnahmewinkel-Paar mit einem kleineren Zwischenwinkel.

6. Verfahren nach einem der vorhergehenden Ansprüche,
**dadurch gekennzeichnet, dass**
das Bewertungskriterium eine zu erwartende Strahlenbelastung eines Patienten, für dessen Körpergefäßsegment (14) die Angiographie erstellt wird, umfasst und eine geringe Strahlenbelastung besser bewertet wird als eine hohe Strahlenbelastung.

7. Verfahren nach einem der vorhergehenden Ansprüche,
**dadurch gekennzeichnet, dass**
das Bewertungskriterium eine zu erwartende Bildqualität der Aufnahme umfasst, insbesondere ein zu erwartendes Signal-zu-Rausch-Verhältnis und/oder eine zu erwartende Bildschärfe, und eine hohe Bildqualität besser bewertet wird als eine geringe Bildqualität.

8. Verfahren nach einem der vorhergehenden Ansprüche,
**dadurch gekennzeichnet, dass**
das Bewertungskriterium eine für die Aufnahmen veränderbare Tischposition eines Patiententisches des Röntgengeräts umfasst, und ein Aufnahmewinkel-Paar ohne eine Veränderung beziehungsweise mit einer geringeren Veränderung der Tischposition besser bewertet wird als ein Aufnahmewinkel-Paar mit einer Veränderung beziehungsweise mit einer größeren Veränderung der Tischposition.

9. Verfahren nach einem der Ansprüche 1 bis 6 oder nach Anspruch 8,
**dadurch gekennzeichnet, dass**
die Tischposition des Patiententisches jeweils für ein Aufnahmewinkel-Paar festgelegt wird, und insbesondere sich das eine Körpergefäßsegment (14) durch das Festlegen in einem Isozentrum des Röntgengerätes befindet.

10. Verfahren nach einem der vorhergehenden Ansprüche,
**dadurch gekennzeichnet, dass**
der in der Ebene senkrecht zu der Rotationsachse (16) von den Aufnahmewinkeln eingeschlossene Zwischenwinkel zwischen 25° und 90° beträgt, bevorzugt zwischen 30° und 90°.

11. Verfahren nach einem der vorhergehenden Ansprüche,
**dadurch gekennzeichnet, dass**
das Auswählen (7) automatisch durch die Recheneinrichtung des Röntgengerätes erfolgt.

12. Verfahren nach einem der vorhergehenden Ansprüche,
**dadurch gekennzeichnet, dass**
die bewerteten Aufnahmewinkel von Schritt d) jeweils senkrecht zu der Rotationsachse (16) stehen.

13. Röntgengerät für ein Erstellen (8) einer dreidimensionalen Angiographie eines Körpergefäßsegments (14), mit einer Recheneinrichtung, an welche eine dreidimensionale Rekonstruktion (10) des Körpergefäßsegments (14) bereitstellbar ist, wobei durch die Recheneinrichtung eine Mittellinie (15) durch das Körpergefäßsegment (14) berechenbar und eine Rotationsachse (16) durch die Mittellinie (15) legbar ist, sowie die dreidimensionale Rekonstruktion (10) zu dem Röntgengerät registrierbar ist, und die Recheneinrichtung ausgelegt ist, die Eignung von zumindest einem Aufnahmewinkel-Paar mit einem ersten und einem zweiten Aufnahmewinkel für das Erstellen (8) der dreidimensionalen Angiographie anhand eines Bewertungskriteriums zu bewerten, wobei die bewerteten Aufnahmewinkel jeweils senkrecht zu der Rotationsachse (16) stehen, und eines des zumindest einen Aufnahmewinkel-Paares mit einem der ersten und einem der zweiten Aufnahmewinkel zum Erstellen (8) der dreidimensionalen Angiographie in Abhängigkeit eines Ergebnisses des Bewertens auszuwählen.

## Claims

1. Method for operating an x-ray device for creation (8) of a three-dimensional angiography of a body vessel segment (14), with the steps:
a) Provision (1) of a three-dimensional reconstruction (10) of the body vessel segment (14) to a computing device of the x-ray device;
b) Calculation (3) of a centre line (15) through the body vessel segment (14) and laying (4) of an axis of rotation (16) through the centre line (15) by means of the computing device;
c) Registration (5) of the three-dimensional reconstruction (10) with the x-ray device;
d) Evaluation (6) of the suitability of at least one recording angle pair with a first and a second recording angle for the creation (8) of the three-dimensional angiography on the basis of an assessment criterion by means of the computing device;
e) Selection (7) of one of the at least one assessed recording angle pairs for creation (8) of the three-dimensional angiography as a function of a result of the assessment.

2. Method according to claim 1,
**characterised in that**
the assessment criterion comprises a degree of a superimposition of at least one further body vessel segment (17) on the body vessel segment (14) on the respective recording of the one body vessel segment (14), and a lower degree of superimposition is assessed as being better than a higher degree of superimposition.

3. Method according to one of the preceding claims,
**characterised in that**
the assessment criterion comprise a degree of a peripheral superimposition of at least one further body vessel segment (17) with each other on the respective recording of the one body vessel segment (14), and a smaller degree of superimposition is assessed as being better than a high degree of superimposition, wherein in particular the degree of the peripheral superimposition will be taken into account less in the result of the assessment than the degree of the superimposition for the one body vessel segment (14).

4. Method according to one of the preceding claims,
**characterised in that**
the assessment criterion comprises an accessibility of the corresponding recording angle by the x-ray device, and a recording angle not able to be accessed by the x-ray device cannot be selected for creation (8) of the three-dimensional angiography, and also in particular a recording angle pair accessible with a lower movement effort will be assessed as being better than a recording angle pair accessible with a higher movement effort.

5. Method according to one of the preceding claims,
**characterised in that**
the recording angles, in a plane perpendicular to the axis of rotation (16), enclose a predetermined intermediate angle and the assessment criterion comprise the intermediate angle, and a recording angle pair with a larger intermediate angle is assessed as being better than a recording angle pair with a smaller intermediate angle.

6. Method according to one of the preceding claims,
**characterised in that**
the assessment criterion comprises a radiation load to be expected for a patient, for whose body vessel segment (14) the angiography will be created, and a small radiation load is assessed as better than a high radiation load.

7. Method according to one of the preceding claims,
**characterised in that**
the assessment criterion comprises an image quality of the recording to be expected, in particular a signal-to-noise ratio and or an image sharpness to be expected, and a high image quality is assessed as being better than a low image quality.

8. Method according to one of the preceding claims,
**characterised in that**
the assessment criterion comprises a position of a patient table of the x-ray device able to be changed for the recording, and a recording angle pair without a change or with a small change of the table position is assessed as being better than a recording angle pair with a change or with a larger change of the table position.

9. Method according to one of claims 1 to 6 or according to claim 8,
**characterised in that**
the table position of the patient table is defined in each case for a recording angle pair, and in particular the one body vessel segment (14) is located by the definition in an isocentre of the x-ray device.

10. Method according to one of the preceding claims,
**characterised in that**
the intermediate angle enclosed in the plane perpendicular to the axis of rotation (16) by the recording angles amounts to between 25° and 90°, preferably between 30° and 90°.

11. Method according to one of the preceding claims,
**characterised in that**
the selection (7) is made automatically by the computing device of the x-ray device.

12. Method according to one of the preceding claims,
**characterised in that**
the assessed recording angles of step d) are perpendicular to the axis of rotation (16) in each case.

13. X-ray device for a creation (8) of a three-dimensional angiography of a body vessel segment (14), with a computing device, on which a three-dimensional reconstruction (10) of the body vessel segment (14) is able to be provided, wherein a centre line (15) through the body vessel segment (14) is able to be computed by the computing device and an axis of rotation (16) is able to be laid through the centre line (15), and also the three-dimensional reconstruction (10) is able to be registered with the x-ray device, and the computing device is designed to evaluate the suitability of at least one recording angle pair with a first and a second recording angle for the creation (8) of the three-dimensional angiography on the basis of an assessment criterion, wherein the assessed recording angles are perpendicular to the axis of rotation (16) in each case, and to select one of the at least one recording angle pairs with one of the first and one of the second recording angles for creation (8) of the three-dimensional angiography as a function of a result of the assessment.

## Revendications

1. Procédé pour faire fonctionner un appareil à rayons X de production (8) d'une angiographie en trois dimensions d'un segment (14) de vaisseau corporel, comprenant les stades :
a) on se procure (1) une reconstruction (10) en trois dimensions du segment (14) de vaisseau corporel sur un dispositif de calcul de l'appareil à rayons X.
b) on calcule (3) une ligne (15) médiane du segment (14) de vaisseau corporel et on fait passer (4) un axe (16) de rotation par la ligne (15) médiane au moyen du dispositif de calcul;
c) on enregistre (5) la reconstruction (10) en trois dimensions dans l'appareil à rayons X;
d) on évalue (6) l'aptitude d'au moins une paire d'angles de prise de vue ayant un premier et un deuxième angles de prise de vue à la production (8) de l'angiographie en trois dimensions, à l'aide d'un critère d'évaluation, au moyen du dispositif de calcul;
e) on choisit (7) l'une des au moins une paire d'angles de prise de vue évaluée pour produire (8) l'angiographie en trois dimensions, en fonction d'un résultat de l'évaluation.

2. Procédé suivant la revendication 1,
**caractérisé en ce que**
le critère d'évaluation comprend un degré d'une superposition du segment (14) de vaisseau corporel à au moins un autre segment (17) de vaisseau corporel sur l'enregistrement respectif du un segment (4) de vaisseau corporel et on évalue, comme meilleur, un degré de superposition plus petit qu'un degré de superposition plus grand.

3. Procédé suivant l'une des revendications précédentes,
**caractérisé en ce que**
le critère d'évaluation comprend un degré d'une superposition périphérique au moins d'un autre segment (17) de vaisseau corporel entre eux sur l'enregistrement respectif du un segment (14) de vaisseau corporel et on évalue, comme meilleur, un degré de superposition plus petit qu'un degré de superposition plus grand, dans lequel on tient moins compte notamment du degré d'une superposition périphérique dans le résultat de l'évaluation que du degré de la superposition pour le segment (14) de vaisseau corporel.

4. Procédé suivant l'une des revendications précédentes,
**caractérisé en ce que**
le critère d'évaluation comprend une possibilité de mise en oeuvre de l'angle de prise de vue correspondant par l'appareil à rayons X et un angle de prise de vue, ne pouvant pas être mis en oeuvre par l'appareil de rayons X, ne peut pas être choisi pour la production (8) de l'angiographie en trois dimensions, ainsi que, notamment, une paire d'angles de prise de vue pouvant être mise en oeuvre avec une complexité de mouvement plus petite, est mieux évaluée qu'une paire d'angles de prise de vue pouvant être mise en oeuvre avec une complexité de déplacement plus grande.

5. Procédé suivant l'une des revendications précédentes,
**caractérisé en ce que**
l'angle de prise de vue fait, dans un plan perpendiculaire à l'axe (6) de rotation, un angle intermédiaire donné à l'avance et le critère d'évaluation comprend l'angle intermédiaire et on évalue mieux une paire d'angles de prise de vue ayant un angle intermédiaire plus grand qu'une paire d'angles de prise de vue ayant un angle intermédiaire plus petit.

6. Procédé suivant l'une des revendications précédentes,
**caractérisé en ce que**
le critère d'évaluation comprend une charge de rayonnement escomptée d'un patient pour le segment (14) de vaisseau corporel duquel on produit l'angiographie et on évalue mieux une charge de rayonnement plus petite qu'une charge de rayonnement plus grande.

7. Procédé suivant l'une des revendications précédentes,
**caractérisé en ce que**
le critère d'évaluation comprend une qualité d'image escomptée de l'enregistrement, notamment un rapport signal à bruit escompté et/ou une netteté d'image escomptée et on évalue mieux une meilleure qualité d'image qu'une qualité d'image moins bonne.

8. Procédé suivant l'une des revendications précédentes,
**caractérisé en ce que**
le critère d'évaluation comprend une position, pouvant être modifiée pour les enregistrements, d'une couchette de patient de l'appareil à rayons X et on évalue mieux une paire d'angles de prise de vue sans modification ou avec une modification plus petite de la position de la couchette qu'une paire d'angles de prise de vue ayant une modification ou une modification plus grande de la position de la couchette.

9. Procédé suivant l'une des revendications 1 à 6 ou suivant la revendication 8,
**caractérisé en ce que**
l'on fixe la position de la couchette du patient respectivement pour une paire d'angles de prise de vue et, notamment, le un segment (14) de vaisseau corporel se trouve, par la fixation, dans un isocentre de l'appareil à rayons X.

10. Procédé suivant l'une des revendications précédentes,
**caractérisé en ce que**
l'angle intermédiaire fait dans le plan, perpendiculairement à l'axe (16) de rotation par les angles de prise de vue, est compris entre 25° et 90°, de préférence entre 30° et 90°.

11. Procédé suivant l'une des revendications précédentes,
**caractérisé en ce que**
le choix (7) s'effectue automatiquement par le dispositif de calcul de l'appareil à rayons X.

12. Procédé suivant l'une des revendications précédentes,
**caractérisé en ce que**
les angles de prise de vue évalués du stade d) sont chacun perpendiculaires à l'axe (16) de rotation.

13. Appareil à rayons X pour la production (8) d'une angiographie en trois dimensions d'un segment (14) de vaisseau corporel, comprenant un dispositif de calcul, sur lequel une reconstruction (10) en trois dimensions du segment (14) de vaisseau corporel peut être mise à disposition, dans lequel, par le dispositif de calcul, une ligne (15) médiane, passant par le segment (14) du vaisseaux corporel, peut être calculée et un axe (16) de rotation peut passer par la ligne (15) médiane, ainsi que la reconstruction (10) en trois dimensions peut être enregistrée dans l'appareil à rayons X et le dispositif de calcul est conçu pour évaluer l'aptitude d'au moins une paire d'angles de prise de vue, comprenant un premier et un deuxième angles de prise de vue, à la production (8) de l'angiographie en trois dimensions, à l'aide d'un critère d'évaluation, les angles de prise de vue évalués étant chacun perpendiculaires à l'axe (16) de rotation et l'une des au moins une paire d'angles de prise de vue, ayant un premier et un deuxième angle de prise de vue, est choisie pour produire (8) l'angiographie en trois dimensions en fonction d'un résultat de l'évaluation.
